# EUROPEAN PATENT SPECIFICATION

(11) **EP 4 386 089 B1**
(45) Date of publication and mention of the grant of the patent: **03.09.2025**
(21) Application number: 22383213.0
(22) Date of filing: 14.12.2022
(51) Int. Cl.: C12Q 1/6883

(54) **METHOD FOR THE DIAGNOSIS OF JOUBERT SYNDROME**
VERFAHREN ZUR DIAGNOSE DES JOUBERT-SYNDROMS
PROCÉDÉ DE DIAGNOSTIC DU SYNDROME DE JOUBERT

(43) Date of publication of application: 19.06.2024
(73) Proprietor: Fundacion Instituto De Investigacion Sanitaria Fundacion Jimenez Diaz, 28040 Madrid (ES)
(72) Inventor: ALMOGUERA CASTILLO, Berta, 28040 Madrid (ES); AYUSO GARCÍA, Carmen, 28040 Madrid (ES); MARTÍNEZ GRANERO, Francisco, 28040 Madrid (ES); LORDA SÁNCHEZ, Isabel, 28040 Madrid (ES); MARTÍNEZ CAYUELAS, Elena, 28040 Madrid (ES); BLANCO KELLY, Fiona, 28040 Madrid (ES); RODRÍGUEZ DE ALBA FREIRÍA, Marta, 28040 Madrid (ES); RODILLA HERNÁNDEZ, Cristina, 28040 Madrid (ES); CORTÓN PÉREZ, Marta, 28040 Madrid (ES); NÚÑEZ MORENO, Gonzalo, 28040 Madrid (ES); ROMERO FERNÁNDEZ, Raquel, 28040 Madrid (ES); MÍNGUEZ PANIAGUA, Pablo, 28040 Madrid (ES)
(74) Representative: Hoffmann Eitle

(56) References cited:
- CN-A- 108 531 580
- BONNARD CARINE ET AL: "Novel mutations in the ciliopathy-associated geneCPLANE1(C5orf42) cause OFD syndrome type VI rather than Joubert syndrome", EUROPEAN JOURNAL OF MEDICAL GENETICS, ELSEVIER, NL, vol. 61, no. 10, 30 March 2018 (2018-03-30), pages 585 - 595, XP085468241, ISSN: 1769-7212, DOI: 10.1016/J.EJMG.2018.03.012
- KIRK EDWIN P ET AL: "Beyond the panel: preconception screening in consanguineous couples using the TruSight One "clinical exome"", GENETICS IN MEDICINE, NATURE PUBLISHING GROUP US, NEW YORK, vol. 21, no. 3, 2 July 2018 (2018-07-02), pages 608 - 612, XP036859030, ISSN: 1098-3600, [retrieved on 20180702], DOI: 10.1038/S41436-018-0082-9
- FEI HONGJUN ET AL: "variants causing Joubert syndrome", MOLECULAR GENETICS & GENOMIC MEDICINE, vol. 10, no. 3, 29 January 2022 (2022-01-29), XP093044581, ISSN: 2324-9269, Retrieved from the Internet <URL:https://onlinelibrary.wiley.com/doi/full-xml/10.1002/mgg3.1877> DOI: 10.1002/mgg3.1877
- ZHANG CHENG ET AL: "Novel compound heterozygous CPLANE1 variants identified in a Chinese family with Joubert syndrome", INTERNATIONAL JOURNAL OF DEVELOPMENTAL NEUROSCIENCE., vol. 81, no. 6, 25 June 2021 (2021-06-25), GB, pages 529 - 538, XP093044582, ISSN: 0736-5748, Retrieved from the Internet <URL:https://onlinelibrary.wiley.com/doi/full-xml/10.1002/jdn.10135> DOI: 10.1002/jdn.10135
- MARTÍNEZ-GRANERO FRANCISCO ET AL: "Biallelic intragenic tandem duplication of CPLANE1 in Joubert syndrome: A case report", CLINICAL GENETICS, vol. 103, no. 4, 31 January 2023 (2023-01-31), DK, pages 448 - 452, XP093044579, ISSN: 0009-9163, Retrieved from the Internet <URL:https://onlinelibrary.wiley.com/doi/full-xml/10.1111/cge.14306> DOI: 10.1111/cge.14306

## Description

### FIELD OF THE INVENTION

The present invention refers to the medical field. Particularly, the present invention refers to an *in vitro* method for the diagnosis of Joubert syndrome (JS) by assessing the presence of a biallelic intragenic duplication of exons 20-46 of gene *CPLANE1.*

### STATE OF THE ART

JS is a rare genetic disorder characterized by a distinctive magnetic resonance imaging (MRI) finding: the molar tooth sign (MTS). It is defined by an abnormally deep interpeduncular fossa; elongated, thick, and mal-oriented superior cerebellar peduncles; and absent or hypoplastic cerebellar vermis that together give the appearance of a "molar tooth" on axial brain MRI. JS is a ciliopathy, which results from the dysfunction of the primary cilium and is associated with multi-organ involvement.

To date, 40 JS-causing genes have been reported, all of them with autosomal recessive inheritance except *OFD1,* with an X-linked pattern, and *SUFU,* with an apparent dominant inheritance and reduced penetrance. *CPLANE1* is one of the most frequently mutated genes, with pathogenic sequence variants explaining up to 14% of the cases. Most of such variants are truncating and missense, and, to date eight intragenic duplications have been reported in ClinVar, with only two classified as likely pathogenic. CN 108 531 580 A discloses a method of screening a biological sample suffering from Joubert syndrome wherein the presence of point mutations in the C5orf42 gene is an indication that the biological sample is suffering from Joubert syndrome. BONNARD CARINE ET AL (2018) relates to the association CPLANE1 mutations with Joubert disease.

KIRK EDWIN P ET AL (2018) relates to the association *CPLANE1* variant with Joubert disease.

FEI HONGJUN ET AL (2022) relates to the association CPLANE1 variant with Joubert disease. ZHANG CHENG ET AL (2021) relates to novel heterozygous CPLANE1 variants identified in a Chinese family with Joubert syndrome.

So, there is an unmet medical need of finding effective tools for the diagnosis of JS. The present invention is focused on solving this problem and a new method is herein provided.

### DESCRIPTION OF THE INVENTION

### Brief description of the invention

The present invention refers to an *in vitro* method for the diagnosis of JS. Particularly, the inventors of the present invention have identified a novel biallelic intragenic duplication of exons 20-46 of gene *CPLANE1.* The quadruplication was identified by short-read sequencing and copy number variant analysis. An array CGH was used to prove the parental origin the variant. The duplication was confirmed in tandem by long PCR and the break points were defined by a nanopore-based long-read sequencing approach. Based on the genetic findings and the clinical presentation of the patient, a brain MRI was ordered, evidencing the molar tooth sign, which confirmed the diagnosis of JS in the patient.

Consequently, the inventors of the present invention have identified for the first time the association of an intragenic duplication in gene *CPLANE1* with JS. In other words, the inventors of the present invention have described the first case of JS caused by a biallelic intragenic duplication of CPLANE1, identified by short and long-reads sequencing approaches and confirmed in tandem. A postnatal MRI was performed at four months of age but there was no record in her medical history of the presence of the MTS. It was only upon identification of the *CPLANE1* duplication and with a specific suspicion of JS, that a new MRI was ordered and evidenced the malformation. This result shows that the genetic study is critical in the characterization of patients with JS with no reported or apparent MRI abnormalities.

The inventors of the present invention identified the duplication of exons 20 to 46 of *CPLANE1* using short-read sequencing with CNV analysis. They used a long-read nanopore-based approach to locate the exact breakpoints. The results highlight the importance of screening for non-conventional variants such as structural or intronic variants in JS as well as in other Mendelian disorders, to increase the diagnostic yield.

The inventors of the present invention evidenced that the duplication was in tandem, confirming the genomic coordinates of the break points and the exact size of the region involved. The duplication was in direct orientation and in-frame, so a lengthened protein with 1,763 additional amino acids with a potential deleterious effect on the protein is expected. The duplication break points are intronic, and although the potential effect on the splicing is difficult to ascertain, the creation of alternative donor or acceptor splicing sites with a loss-of-function effect of the variant cannot be discarded.

So, the first embodiment of the present invention refers to an *in vitro* method for the diagnosis of JS which comprises assessing the presence of a biallelic intragenic duplication of exons 20-46 of gene *CPLANE1* in a biological sample obtained from the subject, wherein the presence of a biallelic intragenic duplication of exons 20-46 of gene *CPLANE1* is an indication that the subject may be suffering from JS.

In a preferred embodiment, the method further comprises confirming the diagnosing of Joubert syndrome by assessing the presence of the molar tooth sign by using an imaging technique.

In a preferred embodiment, the method further comprises confirming the diagnosing of Joubert syndrome by assessing the presence of the molar tooth sign by magnetic resonance imaging (MRI).

In a preferred embodiment, the biological sample is DNA from peripheral blood, serum or plasma samples.

The second embodiment of the present invention refers to the *in vitro* use of the presence of a biallelic intragenic duplication of exons 20-46 of gene *CPLANE1* for the diagnosis of JS.

In a preferred embodiment, the present invention refers to the *in vitro* use of the presence of a biallelic intragenic duplication of exons 20-46 of gene *CPLANE1,* in combination with an imaging technique, for the diagnosis of JS.

In a preferred embodiment, the present invention refers to the *in vitro* use of the presence of a biallelic intragenic duplication of exons 20-46 of gene *CPLANE1,* in combination with a magnetic resonance imaging, for the diagnosis of JS.

Also disclosed but not part of the invention is a method for detecting a biallelic intragenic duplication of exons 20-46 of gene *CPLANE1* in a test sample from a human subject at risk of developing JS, the method comprising: a) Contacting the test sample with primers specific to the biomarker, wherein the biomarker comprises the biallelic intragenic duplication of exons 20-46 of gene *CPLANE1*; b) amplifying the biomarker to produce an amplification product in the test sample; and c) determining the level of the amplification product in the test sample.

For the purpose of the present invention the following terms are defined:
- The term "comprising" means including, but not limited to, whatever follows the word "comprising". Thus, use of the term "comprising" indicates that the listed elements are required or mandatory, but that other elements are optional and may or may not be present.
- The term "consisting of" means including, and limited to, whatever follows the phrase "consisting of". Thus, the phrase "consisting of" indicates that the listed elements are required or mandatory, and that no other elements may be present.

### Description of the figures

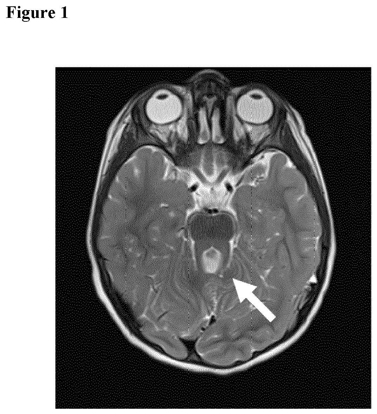

**Figure 1****.** Axial brain MRI image of the patient evidencing the MTS and confirming the diagnosis of JS.

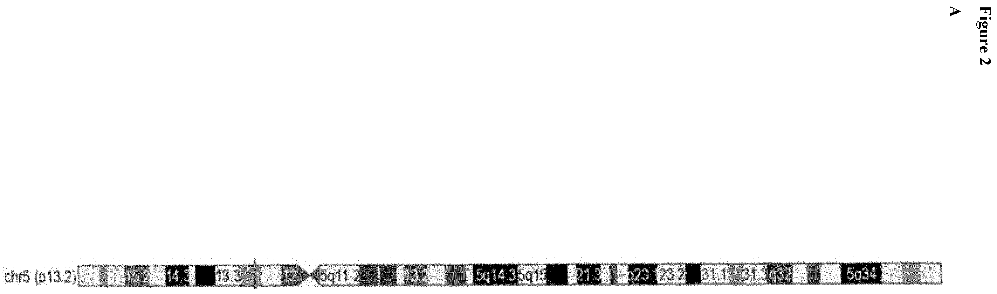

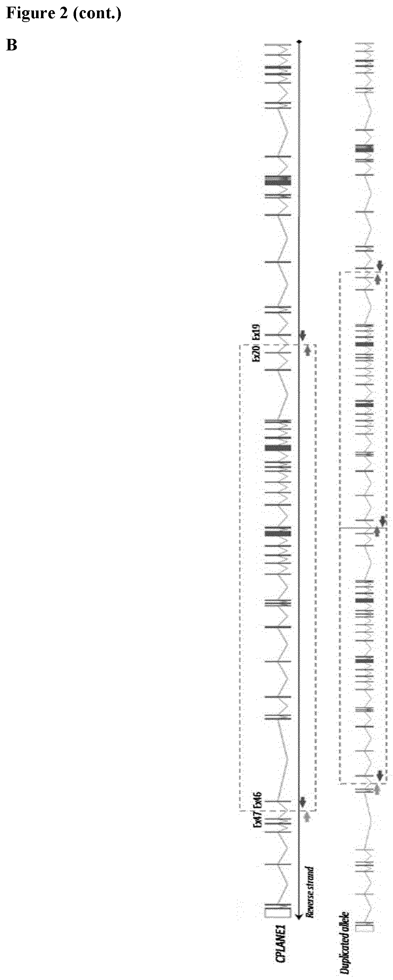

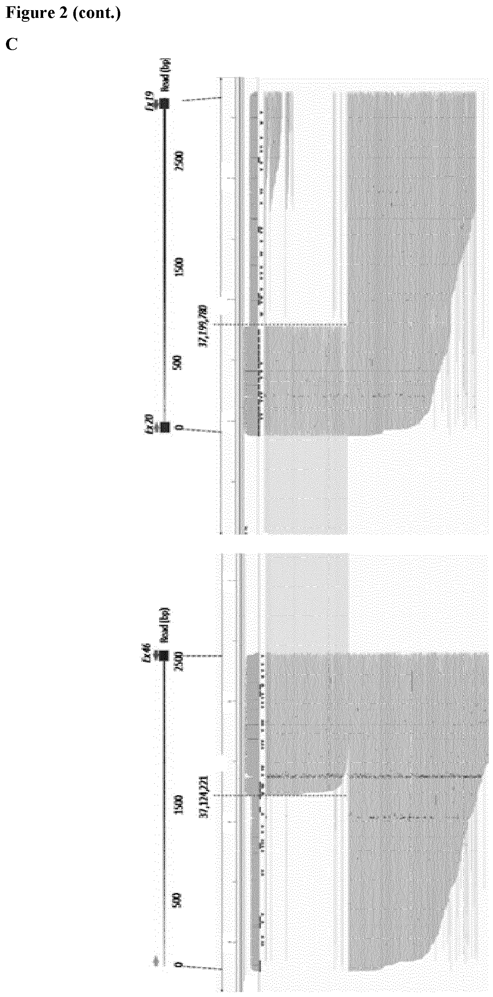

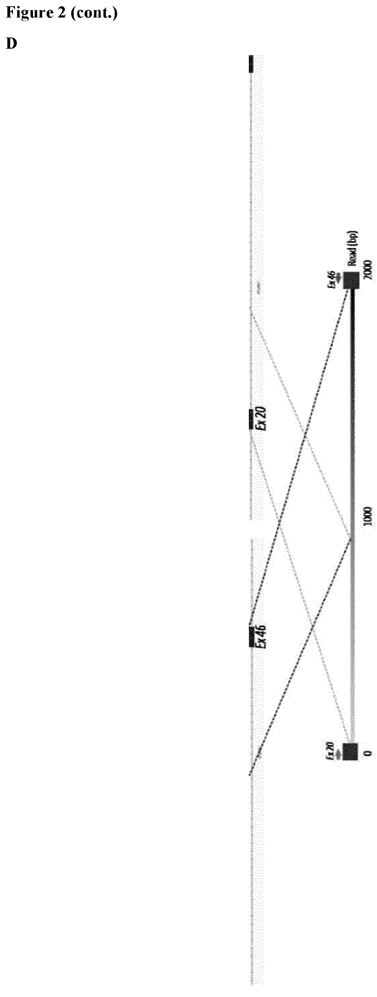

**Figure 2****.** Fine mapping of the duplication in *CPLANE1* by long-read sequencing. **A.** UCSC database representation of the *CPLANE1* gene on the reverse strand and the duplication detected. **B.** Schematic representation of the intragenic tandem duplication of exons 20-46. Colour arrows represent the primers used to amplify and confirm the tandem duplication by long-PCR and long-read sequencing. **C.** Nanopore-based long-read sequencing confirmed the tandem duplication and delimited the breakpoint junctions. Integrative Genomics Viewer (IGV) screenshots of the long-read mapping for the proximal and distal breakpoints on intron 46 and intron 19, respectively. Gene, read coverage, and alignment tracks are shown from top to bottom. Reads are colored by pair orientation and linked by supplementary alignment (the thin lines between both sides). **D.** Schematic representation for the structure and mapping of a chimeric long read for the duplication.

### Detailed description of the invention

The present invention is illustrated by means of the Examples set below without the intention of limiting its scope of protection.

### Example 1. Patient and clinical history

The case is a 3-year-old female patient, the first child of a non-consanguineous healthy couple from Mexico, born after an unremarkable pregnancy. In the fourth month of pregnancy, cerebellar vermis hypoplasia was suspected by routine ultrasound; however, no prenatal MRI was performed. An MRI performed at four months of age showed a moderate increase in the size of the basal cisternae, and subarachnoid spaces in the convexity, without alteration of the vermis or cerebellum. At 10 months, the patient was again referred to the neurologist due to abnormal stereotypic movements.

At 15 months of age, the patient was evaluated at the Pediatric Neurology Department of Fundación Jiménez Diaz (FJD-UH) due to developmental delay and generalized hypotonia. She presented dysmorphic facial features (large eyes, downslanting palpebral fissures, anteverted nares, and bushy eyebrows) and an inguinal hernia. Respiratory, cardiovascular, neurological, and abdominal examinations, as well as urine quantification of mucopolysaccharides and general blood tests were normal. A clinical exome sequencing study was ordered and upon genetic results, at 27 months of age, the patient was ordered a new MRI that evidenced the MTS **(****Figure 1****).**

### Example 2. Identification and characterization of a tandem duplication in CPLANE1

Short-read sequencing was performed using the SOPHiA Genetics Clinical Exome Solution v2 kit (CES; SOPHiA Genetics, Boston, MA) on a NextSeq500 instrument (Illumina, San Diego, CA). We used the DDM^{™} platform (SOPHiA Genetics) for single nucleotide (SNV) and copy number variants (CNV) analyses with a virtual panel of 764 genes involved in global developmental delay and intellectual disability (see Supplemental Material).

The CES analysis identified a quadruplication of exons 20 to 46 of *CPLANE1* (NM_023073, *RefSeq)* **(****Figures 2****)** (NC_000005.10:g.(37122622_37125336)_(37198978_37201682)[4]) (hg19). To confirm the findings and segregate the duplication in the parents, an array comparative genomic hybridization (aCGH) was performed with the aCGX 60K platform (CGX^{™}, PerkinElmer, Inc), the SureScan Microarray Scanner (Agilent Technologies, Santa Clara, California, USA) and the Genoglyphix^{®} platform (PerkinElmer, Inc). The aCGH used did not cover the entire sequence presumably duplicated, but the log2 ratio values of the probes confirmed four copies in the patient and three in the parents.

The duplication was absent in population genomic databases (gnomAD Structural Variants, 1000 Genomes Project, and the Database of Genomic Variants) and in our internal database of 1,889 patients with CES data. Eight intragenic duplications were reported in ClinVar involving different regions of *CPLANE1,* six classified as variants of uncertain significance (VUS) and two as likely pathogenic (accessed on October 6^{th}, 2022). No duplications of *CPLANE1* were reported in the Human Genetic Mutation Database (HGMD, professional version, accessed on October 6th, 2022) or PubMed, and duplications reported in DECIPHER were larger and included other genes besides *CPLANE1.* Thus, the duplication was classified as VUS.

We investigated whether the intragenic duplication was in tandem. Considering that tandem duplications are mainly located in direct orientation, we designed an assay including three long PCRs using the Expand High Fidelity PCR System (Roche Diagnostics GmbH). A specific primer in exon 46 and a reverse primer in exon 20 were designed (Supplemental Material), so a PCR product could only amplify if direct tandem duplication was present. The PCR yielded the same amplicon of approximately 2300bp in the patient and both parents and no amplification in the control DNA. Two additional PCRs were designed to amplify introns 19 and 46, respectively, to allow the identification of the breakpoints (Supplemental Material). Long-read sequencing of the three amplicons (Supplemental Material) allowed for fine mapping of the duplication with proximal and distal junctions located on intron 46 and intron 19 (chr5:37124221-37199780; hg19) **(****Figure 2****)** and a size of 75,559 bp.

The evidence of the biallelic duplication of 76.5Kb in tandem, involving 1,763 amino acids, with a likely deleterious effect on the protein; the parental origin of the duplication; and the presence of the MTS on MRI, support a potential pathogenic effect of the variant.

### Example 3. Ancestral origin of the duplication

We explored the presence of a common haplotype in the parents. Both parents were Mexican, but with ancestors from Cantabria, a region in Northern Spain. Haplotype analysis included two short tandem repeat (STR) markers upstream (D5S2085 and DS1994 at 0.76Mb and 0.64Mb from *CPLANE1,* respectively) and two downstream (D5S2023 and D5S1998, at 0.24Mb and 0.48Mb from *CPLANE1,* respectively). The patient was heterozygous for three STRs, which was not compatible with the duplication arising from a common haplotype.

To complement the haplotype analysis, homozygosity mapping was performed using the CES data of the patient and the software AutoMap (https://automap.iob.ch/), which accurately identifies runs of homozygosity (ROH) from Whole Exome Sequencing data⁹. No ROH was evident in the region flaking or close to *CPLANE1.* However, due to the location of the STR markers used for the haplotyping and the uneven coverage of the CES across the genome, we explored whether there were homozygous rare genetic variants in genes flanking *CPLANE1.* We found two rare homozygous variants in *NIBPL,* a gene located upstream *CPLANE1* and downstream DS1994 (rs398124464 and rs80358355 with MAF of 0.0083 and 0.0174, respectively). These results support the duplication being in a common haplotype smaller than the resolution of the haplotype analysis and homozygosity mapping performed.

## Claims

1. *In vitro* method for the diagnosis of Joubert syndrome which comprises assessing the presence of a biallelic intragenic duplication of exons 20-46 of gene *CPLANE1* in a biological sample obtained from the subject, wherein the presence of a biallelic intragenic duplication of exons 20-46 of gene *CPLANE1* is an indication that the subject may be suffering from Joubert syndrome.

2. *In vitro* method, according to claim 1, wherein the method further comprises confirming the diagnosing of Joubert syndrome by assessing the presence of the molar tooth sign by using an imaging technique.

3. *In vitro* method, according to any of the previous claims, wherein the method further comprises confirming the diagnosing of Joubert syndrome by assessing the presence of the molar tooth sign by magnetic resonance imaging.

4. *In vitro* use of the presence of a biallelic intragenic duplication of exons 20-46 of gene *CPLANE1* for the diagnosis of Joubert syndrome.

5. *In vitro* use of the presence of a biallelic intragenic duplication of exons 20-46 of gene *CPLANE1* for the diagnosis of Joubert syndrome, according to claim 4, in combination with an imaging technique.

6. *In vitro* use of the presence of a biallelic intragenic duplication of exons 20-46 of gene *CPLANE1* for the diagnosis of Joubert syndrome, according to any of the claims 4 or 5, in combination with magnetic resonance imaging.

## Patentansprüche

1. In-vitro-Verfahren zur Diagnose des Joubert-Syndroms, welches das Beurteilen der Anwesenheit einer biallelischen intragenetischen Duplikation der Exonen 20-46 des Gens *CPLANE1* in einer biologischen Probe erhalten aus dem Individuum umfasst, wobei die Anwesenheit einer biallelischen intragenetischen Duplikation der Exonen 20-46 des Gens *CPLANE1* ein Hinweis darauf ist, dass das Individuum unter dem Joubert-Syndrom leiden könnte.

2. In-vitro-Verfahren nach Anspruch 1, wobei das Verfahren zusätzlich das Bestätigen der Diagnose des Joubert-Syndroms umfasst, indem die Anwesenheit des Backenzahnzeichens unter Verwendung einer bildgebenden Technik beurteilt wird.

3. In-vitro-Verfahren nach einem der vorhergehenden Ansprüche, wobei das Verfahren zusätzlich das Bestätigen der Diagnose des Joubert-Syndroms umfasst, indem die Anwesenheit des Backenzahnzeichens mittels Magnetresonanzbildgebung beurteilt wird.

4. In-vitro-Verwendung der Anwesenheit einer biallelischen intragenetischen Duplikation der Exonen 20-46 des Gens *CPLANE1* zur Diagnose des Joubert-Syndroms.

5. In-vitro-Verwendung der Anwesenheit einer biallelischen intragenetischen Duplikation der Exonen 20-46 des Gens *CPLANE1* zur Diagnose des Joubert-Syndroms nach Anspruch 4, in Kombination mit einer bildgebenden Technik.

6. In-vitro-Verwendung der Anwesenheit einer biallelischen intragenetischen Duplikation der Exonen 20-46 des Gens *CPLANE1* zur Diagnose des Joubert-Syndroms nach einem der Ansprüche 4 oder 5, in Kombination mit Magnetresonanzbildgebung.

## Revendications

1. Procédé *in vitro* de diagnostic du syndrome de Joubert qui comprend l'évaluation de la présence d'une duplication intragénique biallélique des exons 20-46 du gène *CPLANE1* dans un échantillon biologique obtenu à partir du sujet, dans lequel la présence d'une duplication intragénique biallélique des exons 20-46 du gène *CPLANE1* est une indication que le sujet peut souffrir du syndrome de Joubert.

2. Procédé *in vitro,* selon la revendication 1, dans lequel le procédé comprend en outre la confirmation du diagnostic du syndrome de Joubert par l'évaluation de la présence du signe de la dent molaire en utilisant une technique d'imagerie.

3. Procédé *in vitro,* selon l'une quelconque des revendications précédentes, dans lequel le procédé comprend en outre la confirmation du diagnostic du syndrome de Joubert par l'évaluation de la présence du signe de la dent molaire par l'imagerie par résonance magnétique.

4. Utilisation *in vitro* de la présence d'une duplication intragénique biallélique des exons 20-46 du gène *CPLANE1* pour le diagnostic du syndrome de Joubert.

5. Utilisation *in vitro* de la présence d'une duplication intragénique biallélique des exons 20-46 du gène *CPLANE1* pour le diagnostic du syndrome de Joubert, selon la revendication 4, en combinaison avec une technique d'imagerie.

6. Utilisation *in vitro* de la présence d'une duplication intragénique biallélique des exons 20-46 du gène *CPLANE1* pour le diagnostic du syndrome de Joubert, selon l'une quelconque des revendications 4 ou 5, en combinaison avec l'imagerie par résonance magnétique.
